# EUROPÄISCHE PATENTANMELDUNG

(11) **EP 2 484 306 A1**
(43) Veröffentlichungstag der Anmeldung: **08.08.2012**
(21) Anmeldenummer: 11000804.2
(22) Anmeldetag: 02.02.2011
(51) Int. Cl.: A61F 2/00, A61F 2/08

(54) **Dauerelastisches Implantat**

(71) Anmelder: AMI Agency for Medical Innovations GmbH, 6800 Feldkirch (AT)
(72) Erfinder: Egle, Walter, 6842 Koblach (DE)

(57) **Zusammenfassung**

Mit einem medizinischen Implantat bestehend aus nichtelastischen, großporigen Abschnitten (3) aus Netzstrukturen, die vom Körpergewebe des Patienten durchwachsen werden und aus dauerelastischen Abschnitten (2), die vom Körpergewebe des Patienten eingekapselt werden, werden chirurgische Rekonstruktionen von Weichteildefekten und chirurgische Repositionierungen von Organen ermöglicht, bei denen die anatomisch gewünschte Elastizität der Rekonstruktion bzw. der Repositionierung erhalten bleibt.

## Beschreibung

Die Erfindung bezieht sich auf ein medizinisches Implantat zur Rekonstruktion von Weichteilstrukturen, wie z.B. Ligamenten oder Faszien- und Muskelblättern, im Rahmen von chirurgischen Eingriffen am menschlichen oder tierischen Körper.

Medizinische Implantate zur Rekonstruktion von Weichteilstrukturen sind in unterschiedlichen Ausführungsformen bekannt und werden üblicherweise als Netz- oder Schlingenimplantate bezeichnet. In älteren und nach wie vor gebräuchlichen Ausführungsformen bestehen solche Implantate aus einem bioinerten, textilen, flächigen Gewebe unterschiedlicher Größe, welches in die zu verstärkende Weichteilstruktur eingelegt wird und in weiterer Folge im umliegenden Gewebe einwächst. Dabei kommt es typischerweise zu einer Narbenbildung rund um die textile Struktur des Implantates, wodurch die anatomisch notwendige Elastizität des Körpergewebes in diesem Bereich stark reduziert oder gar eliminiert wird. Dies führt zu Einschränkungen bis zum Versagen der Funktion von derart chirurgisch rekonstruierten Weichteilstrukturen. Deshalb können solche flächigen, textilen Gewebeimplantate nur beschränkt oder überhaupt nicht eingesetzt werden, wenn der Erhalt der anatomisch vorgesehenen Gewebeelastizität unbedingt notwendig ist, obwohl die Implantate nachweislich für eine effektive, dauerhafte Verstärkung des geschwächten Weichteilgewebes sorgen. Zur chirurgischen Rekonstruktion der verschiedenen Muskel- und Faszienblätter im weiblichen kleinen Becken werden die heute bekannten flächigen, textilen Gewebe deshalb nur bei älteren Patientinnen und sehr ausgeprägten Krankheitsbildern von Organvorfällen eingesetzt. Chirurgen scheuen sich davor bei jüngeren Patientinnen eine komplexe elastische Anatomie durch den Einsatz solcher flächigen, textilen Gewebeimplantate zu versteifen.

Bioinerte, textile Implantate in Bandform, z.B. 1 cm breit und 30 cm lang, werden zur chirurgischen Rekonstruktion von Ligamenten in verschiedenen Regionen des Körpers verwendet. Als Ligamente werden die anatomischen Aufhängungen von Organen oder Muskeln am Skelett bezeichnet. Diese Ligamente zeichnen sich durch hohe Zugfestigkeit und Elastizität aus. Beim chirurgischen Ersatz solcher Ligamente mittels bioinerten, textilen Bändern - in der Chirurgie auch als Schlingen bezeichnet - werden nach dem Verwachsen des Implantates mit dem umliegenden Gewebe hohe Zugfestigkeiten erzielt, die anatomisch geforderte Elastizität allerdings nicht. Deshalb eigenen sich solche textilen Schlingenimplantate nicht oder nur sehr eingeschränkt für die chirurgische Befestigung von dislozierten Organen in deren anatomisch korrekten Position. Als Beispiel sei hier die sogenannte Kolposuspension angeführt, bei welcher der Operateur aufgefordert ist das nach außen vorgefallene Scheidenende wieder in die im Körper innenliegende, anatomisch korrekte Position zu bringen und dort dauerhaft, aber elastisch zu befestigen. Dabei wird z.B. ein Ende der textilen, bioinerten Schlinge am inneren Ende der Scheide angenäht, das Scheidenende in die anatomisch korrekte Position gebracht und das andere Ende der Schlinge am Beckenknochen oder einer anderen geeigneten Stelle mittels Naht oder Klammer befestigt. Dauerhafte, zugfeste Befestigungen dieser Art sind mit den bekannten Ausführungsformen der textilen Schlingenimplantate möglich. Zugfeste und dauerhaft elastische Befestigungen allerdings nicht.

Flächige, bioinerte, textile Netzstrukturen zur chirurgischen Rekonstruktion von Weichteildefekten in der Bauchdecke oder aber im kleinen Becken zur Wiederherstellung der Festigkeit der gewünschten Weichteilstruktur sind in vielfältiger Ausführung bekannt. Eine flächige, textile Netzstruktur, die bei Erhalt der natürlichen Elastizität der Weichteilstruktur eine Wiederherstellung der Festigkeit derselben ermöglicht, ist nicht bekannt.

Aufgabe der Erfindung ist es ein medizinisches Implantat zur Rekonstruktion von Weichteilstrukturen am menschlichen oder tierischen Körper zur Verfügung zu stellen, welches neben einer sicheren, mechanischen Verstärkung der gewünschten Weichteilstruktur deren natürliche Elastizität dauerhaft gewährleistet. Erfindungsgemäß gelingt dies durch ein medizinisches Instrument mit den Merkmalen des Anspruch 1.

Die erfindungsgemäße Ausführung einer solchen textilen, bandförmigen oder flächigen, bioinerten Netzstruktur zur Rekonstruktion von Weichteil- bzw. Ligamentstrukturen besteht aus einer Kombination von dauerelastischen Abschnitten, die vom umgebenden Körpergewebe eingekapselt werden und von nicht elastischen Abschnitten, die vom umgebenden Körpergewebe durchwachsen und in diesem integriert und damit verankert werden. Die vom Körpergewebe durchwachsenen, im Gewebe verankerten Abschnitte des Implantats nehmen die Dehnkräfte, die auf das chirurgisch rekonstruierte oder aber verstärkte Weichteilgewebe wirken, auf und übertragen diese auf die im Körpergewebe nicht verankerten, dauerelastischen Abschnitte des Implantats, die sich im Gewebe den aufzunehmenden Dehnkräften entsprechend verlängern oder verkürzen.

Nachfolgend werden bevorzugte Ausführungsformen der Erfindung unter Bezugnahme auf die beigelegten Zeichnungen beschrieben.

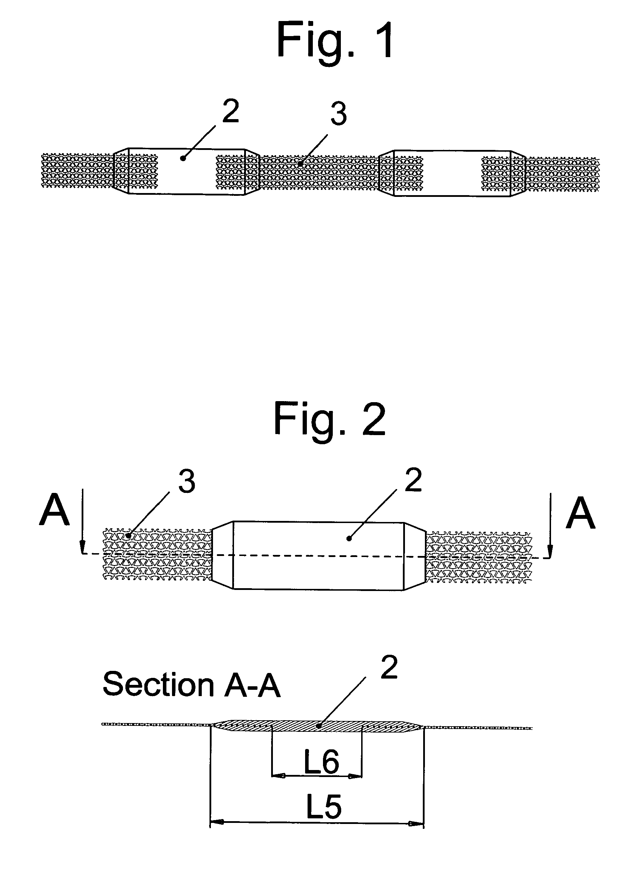

Fig. 1 zeigt ein erfindungsgemäßes, bandförmiges Implantat mit zwei integrierten, dauerelastischen Abschnitten 2, wobei die Anzahl der dauerelastischen Abschnitte 2 mindestens 1 bis ein Vielfaches von 1 sein kann. Die Anzahl der dauerelastischen Abschnitte 2 hängt von der Gesamtlänge des Implantates 1, der Länge des dauerelastischen Abschnittes 2 und der gewünschten Länge der freien, nicht elastischen Verankerungsabschnitte 3 ab.

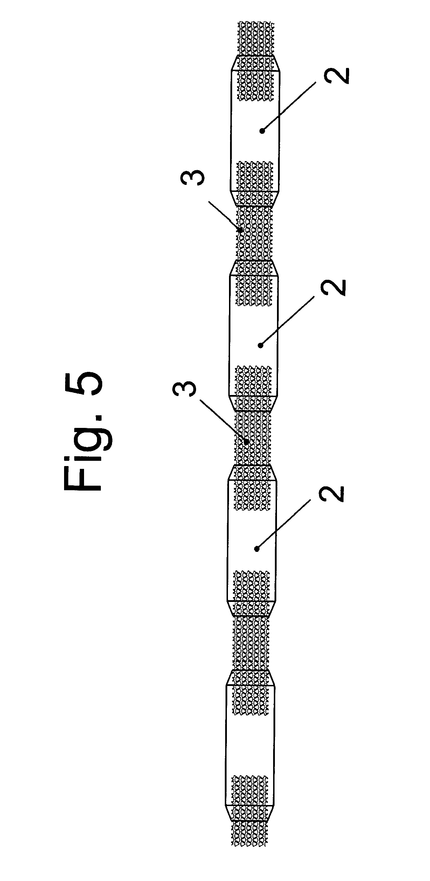

Der Chirurg wird bei einer Rekonstruktion, die postoperativ eine hohe Festigkeit und wenig Zugfestigkeit verlangt, ein Implantat mit vielen Verankerungsabschnittsanteilen 3 und wenig dauerelastischen Abschnitten 2 verwenden und umgekehrt, bei Bedarf nach einer hohen postoperativen Elastizität der chirurgischen Rekonstruktion, ein Implantat mit hohem, dauerelastischen Anteil 2 und wenig Verankerungsabschnittsanteil 3 einsetzen, wie beispielhaft in Fig. 5 gezeigt.

In Fig. 2 wird eine mögliche Ausführungsform des dauerelastischen Abschnittes 2 gezeigt, bei welcher das bioinerte textile Band über eine bestimmte Länge L5 in z.B. Silikon eingelegt ist und die Oberfläche des Implantates über die Länge L5 vollkommen glatt ist und vom Silikon gebildet wird.
Das in Silikon eingelegte, textile Band wird über eine gewünschte Dehnlänge L6 durchtrennt. Die Elastizität des Implantates in Längsrichtung wird durch die Elastizität des verwendeten Silikons, durch die Dehnlänge L6 und die Anzahl der dauerelastischen Abschnitte 2 bestimmt.

Weitere mögliche Ausführungsformen der Verbindung der dauerelastischen Abschnitte mit den nicht elastischen Abschnitten des Implantates können je nach Materialwahl der Abschnitte durch Vernähen, Verkleben, thermisches Verschweißen der Abschnitte miteinander, durch Verkleben, durch Klammern aber auch durch gemeinsames Spritzgießen der Abschnitte erzielt werden.

Als Materialien kommen für die dauerelastischen und nicht elastischen Abschnitte des Implantates alle bioinerten und für Langzeitimplantate zugelassenen Werkstoffe in Frage. Das sind für die dauerelastischen Abschnitte implantatfähige Elastomere und für die nicht elastischen Abschnitte Netzstrukturen mit entsprechenden Poren, die ein Durchwachsen des Implantates vom Körpergewebe ermöglichen, aus textilen oder metallischen Fäden.

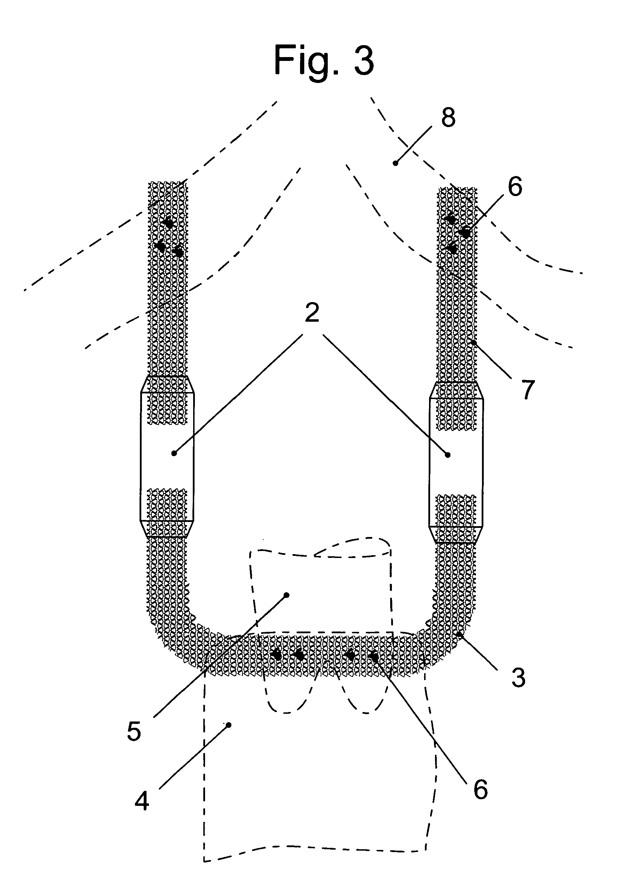

In Fig. 3 wird eine mögliche Ausführungsform eines solchen Implantates gezeigt, wie es zur chirurgischen Repositionierung der Vagina bzw. der Gebärmutter verwendet werden kann. Der nicht elastische Abschnitt 3 des Implantates wird an der Außenwand Vagina 4 bzw. am Gebärmutterhals 5 durch eine chirurgische Naht 6 angenäht und die beiden seitlichen, nicht elastischen Arme 7 des Implantates werden im hinteren Bereich des kleinen Beckens mit dem Sakrospinalen Ligament 8 durch eine chirurgische Naht 6 fest verbunden.
Die Elastizität der chirurgischen Repositionierung von Vagina und Gebärmutter wird durch die beiden elastischen Abschnitte 2 gewährleistet.

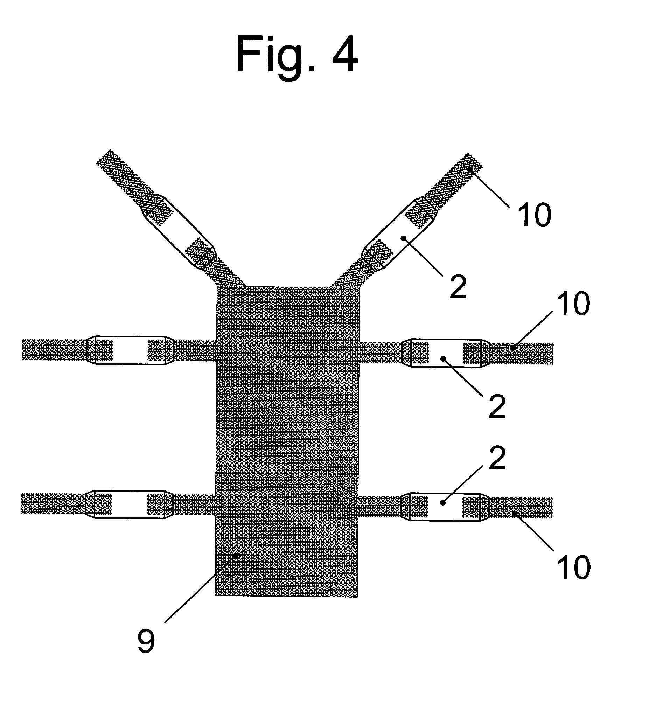

In Fig. 4 wird eine mögliche Ausführung eines solchen Implantates gezeigt, wie es zur chirurgischen Rekonstruktion von Faszien und Muskelschichten im weiblichen Becken verwendet werden kann. Die großflächige, implantatfähige Netzstruktur 9 wird vom Chirurgen in der entsprechenden anatomischen Schicht, z.B. zwischen Harnblase und Vagina, platziert und sorgt nach dem Einwachsen in das körpereigene Gewebe für die gewünschte mechanische Verstärkung dieser Schicht. Die seitlichen Netzarme 10 werden ebenfalls im Gewebe verankert und dienen als Aufhängung für die Netzstruktur 9. Solche Implantate - ohne dauerelastische Abschnitte 2 in den seitlichen Netzarmen 10 - sind bekannt und führen zur gefürchteten und ungewünschten, starren Rekonstruktion des weiblichen Beckenbodens. In der erfindungsgemäßen Ausführungsform werden dauerelastische Abschnitte 2 in die seitlichen Befestigungsarme 10 integriert um eine möglichst, natürliche, dauerelastische Rekonstruktion des Beckenbodens zu ermöglichen.

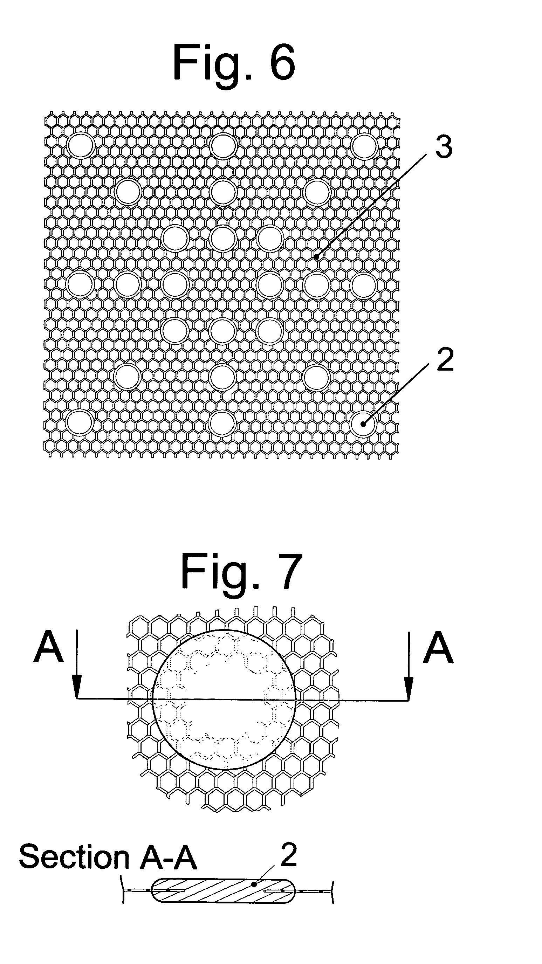

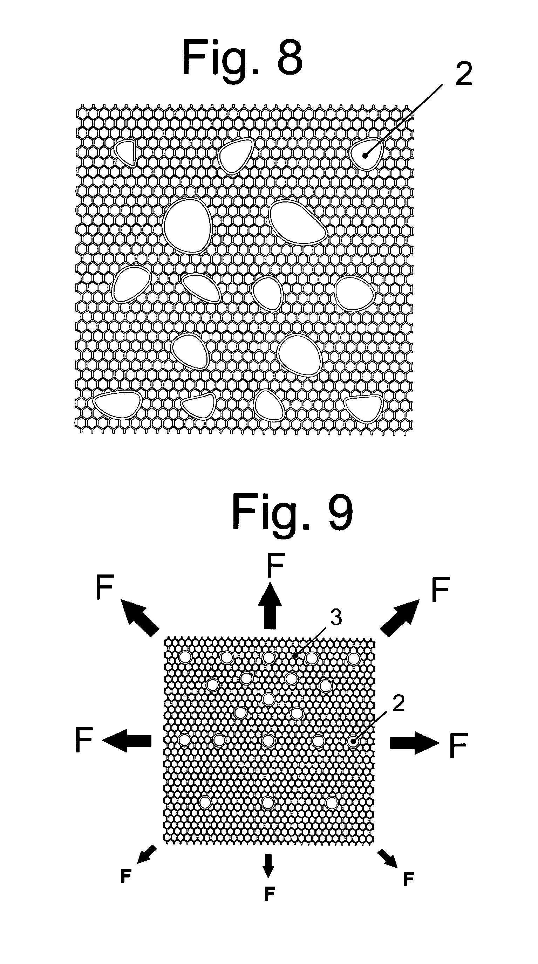

In Fig. 6. wird eine mögliche Ausführung eines solchen Implantates gezeigt, wie es zur Rekonstruktion der Bauchdecke verwendet werden kann. Die dauerelastischen Abschnitte 2 sind im gezeigten Beispiel - siehe Fig. 7 kreisrund ausgeführt und über den nicht elastischen Verankerungsabschnitt 3 gleichmäßig oder aber willkürlich verteilt. Die dauerelastischen Abschnitte 2 können aber ebenso in jeglicher geometrischen Flächenform oder freien Flächenform - wie in Fig. 8 gezeigt, ausgeführt werden.
Wie in Fig. 9 gezeigt, können die integrierten dauerelastischen Abschnitte 2 entsprechend anatomisch angeordnet werden, um je nach Einsatzort in der Bauchdecke den jeweils von unterschiedlichen Richtungen auftretenden unterschiedlichen Dehnkräften F zu entsprechen und mehr oder weniger Gewebeelastizität zur Verfügung stellen.

### Legende zu den Hinweisziffern:

- 1: Implantat
- 2: Dauerelastischer Abschnitt
- 3: Nicht elastischer Verankerungsabschnitt
- 4: Vagina
- 5: Gebärmutterhals
- 6: Chirurgische Naht
- 7: Nichtelastische seitliche Arme des Implantats
- 8: Sakrospinales Ligament
- 9: Großflächiges, implantatfähiges Netz
- 10: Seitliche Netzarme

## Patentansprüche

1. Medizinisches, bandförmiges oder flächiges Implantat aus bioinertem Textilgewebe zur chirurgischen Rekonstruktion von Weichteil- bzw. Ligamentstrukturen, **dadurch gekennzeichnet, dass** ein solches Implantat aus dauerelastischen Abschnitten (2), die mit dem umgebenden Körpergewebe nicht verwachsen und eingekapselt werden und nicht elastischen Abschnitten (3) besteht, die vom umgebenden Körpergewebe durchwachsen und damit im Gewebe fest verankert werden.

2. Medizinisches Implantat nach Anspruch 1, **dadurch gekennzeichnet, dass** die Anzahl der der dauerelastischen Abschnitte (2) mindestens Eins ist oder aber ein Vielfaches von Eins sein kann.

3. Medizinisches Implantat nach Anspruch 1, **dadurch gekennzeichnet, dass** die dauerhafte Längselastizität eines bandförmigen Implantates im Bereich von 0 bis 80%, bevorzugt im Bereich von 5 bis 50% liegt.

4. Medizinisches Implantat nach Anspruch 1, **dadurch gekennzeichnet, dass** die dauerhafte Elastizität eines flächigen Implantates in alle Richtungen im Bereich von 0 bis 60%, bevorzugt im Bereich von 5 bis 35% liegt.

5. Medizinisches Implantat nach Anspruch 1, **dadurch gekennzeichnet, dass** die Form des dauerelastischen Abschnittes beim flächigen Implantat kreisrund, oval, rechteckig, quadratisch, rhombisch oder in jeglicher anderer frei wählbaren geschlossenen Umfangsform ausgeführt ist.

6. Medizinisches Implantat nach Anspruch 1, **dadurch gekennzeichnet, dass** die nicht elastischen Abschnitte aus bioinertem, textilem Netzgewebe aus großporig gewirkten, monofilen Polypropylenfäden bestehen.

7. Medizinisches Implantat nach Anspruch 1, **dadurch gekennzeichnet, dass** die nicht elastischen Abschnitte aus bioinertem Netzgewebe aus großporig gewirkten, monofilen Metallfäden bestehen.

8. Medizinisches Implantat nach Anspruch 1, **dadurch gekennzeichnet, dass** die nicht elastischen Abschnitte aus bioinertem, textilem Netzgewebe aus großporig gewirkten, monofilen Polyesterfäden bestehen.

9. Medizinisches Implantat nach Anspruch 1, **dadurch gekennzeichnet, dass** die nicht elastischen Abschnitte aus bioinertem, textilem Netzgewebe aus großporig gewirkten, multifilen Polypropylen- oder Polyesterfäden bestehen.

10. Medizinisches Implantat nach Anspruch 1, **dadurch gekennzeichnet, dass** die dauerelastischen Abschnitte aus bioinertem, langzeitimplantatfähigem Silikon mit einer Shorehärte im Bereich von 20 bis 80, bevorzugt 30 bis 60 Shore bestehen.

11. Medizinisches Implantat nach Anspruch 1, **dadurch gekennzeichnet, dass** die dauerelastischen Abschnitte aus bioinertem, langzeitimplantatfähigem Polyurethan mit einer Shorehärte im Bereich von 20 bis 80, bevorzugt 30 bis 60 Shore bestehen

12. Medizinisches Implantat nach Anspruch 1, **dadurch gekennzeichnet, dass** die Verbindung der nichtelastischen Abschnitte durch Eingießen der Enden der nicht elastischen Abschnitte in die dauerelastischen Abschnitte erfolgt.

13. Medizinisches Implantat nach Anspruch 1, **dadurch gekennzeichnet, dass** die Verbindung der nichtelastischen Abschnitte des bandförmigen Implantates durch Eingießen der Enden der nicht elastischen Abschnitte in die dauerelastischen Abschnitte erfolgt.

14. Medizinisches Implantat nach Anspruch 1, **dadurch gekennzeichnet, dass** die Länge der nichtelastischen Abschnitte des bandförmigen Implantates im Bereich von 5 bis 200 mm, bevorzugt im Bereich von 10 bis 100 mm liegen.

15. Medizinisches Implantat nach Anspruch 1, **dadurch gekennzeichnet, dass** die Länge der elastischen Abschnitte des bandförmigen Implantates im Bereich von 10 bis 100 mm, bevorzugt im Bereich von 15 bis 60 mm liegen.

16. Medizinisches Implantat nach Anspruch 1, **dadurch gekennzeichnet, dass** der Abstand zwischen den im dauerelastischen Abschnitt eingegossenen Enden des nichtelastischen Abschnitts bei einem bandförmigen Implantat im Bereich von 1 bis 80 mm, bevorzugt im Bereich von 5 bis 50 mm liegt.

17. Medizinisches Implantat nach Anspruch 1, **dadurch gekennzeichnet, dass** die Fläche eines einzelnen dauerhaft elastischen Abschnitts bei einem flächigen Implantat dem Durchmesser eines Kreises im Bereich von 5 bis 50 mm, bevorzugt im Bereich von 10 bis 30mm entspricht.

18. Medizinisches Implantat nach Anspruch 1, **dadurch gekennzeichnet, dass** die Verbindung der nicht elastischen Abschnitte mit den dauerelastischen Abschnitten durch Vernähen derselben miteinander ausgeführt ist.

19. Medizinisches Implantat nach Anspruch 1, **dadurch gekennzeichnet, dass** die Verbindung der nicht elastischen Abschnitte mit den dauerelastischen Abschnitten durch Verkleben derselben miteinander ausgeführt ist.

20. Medizinisches Implantat nach Anspruch 1, **dadurch gekennzeichnet, dass** die Verbindung der nicht elastischen Abschnitte mit den dauerelastischen Abschnitten durch formschlüssiges Verhaken derselben miteinander ausgeführt ist.
